# FASCICULE DE BREVET EUROPEEN

(11) **EP 2 991 732 B1**
(45) Date de publication et mention de la délivrance du brevet: **28.11.2018**
(21) Numéro de dépôt: 14723756.4
(22) Date de dépôt: 29.04.2014
(51) Int. Cl.: A61K 33/30, A61K 33/14, A61K 33/00, A61P 27/04, A61P 27/02, A61P 7/08, C11D 1/00

(54) **SOLUTION MARINE ENRICHIE EN ZINC ET EN POTASSIUM**
ZINK- UND KALIUM-ANGEREICHTE MEERESLÖSUNG
MARINE SOLUTION ENRICHED IN ZINC AND POTASSIUM

(30) Priorité: 03.05.2013 FR 1301040
(43) Date de publication de la demande: 09.03.2016
(73) Titulaire: YS Lab, 29000 Quimper (FR)
(72) Inventeur: HEMON, Marc, 22490 Plouer sur Rance (FR); DUTOT-WOLF, Melody, 94210 La Varenne Saint Hilaire (FR); TANTER, Caroline, 29000 Quimper (FR); FAGON, Roxane, 29140 Saint Yvi (FR)
(74) Mandataire: Ipside
(86) Numéro de dépôt international: PCT/EP2014/058770
(87) Numéro de publication internationale: WO 2014/177594

(56) Documents cités:
- WO-A1-2009/101157
- FR-A1- 2 803 205
- FR-A1- 2 843 029
- MELODY DUTOT: "Effects of toxic cellular stresses and divalent cations on the human P2X7 cell death receptor", MOLECULAR VISION, vol. 14, 1 janvier 2008 (2008-01-01), pages 889-897, XP055070894,
- SAID T ET AL: "Ocular Burn: Rinsing and Healing with Ionic Marine Solutions and Vegetable Oils", OPHTHALMOLOGICA, KARGER, BASEL, CH, vol. 223, 1 janvier 2009 (2009-01-01), pages 52-59, XP009175619, ISSN: 0030-3755 [extrait le 2008-11-20]

## Description

La présente invention concerne une solution à base d'eau de mer, destinée particulièrement à des applications sur les yeux, la peau ou les muqueuses.

Les solutions salines sont couramment utilisées en ophtalmologie en tant qu'excipient pour des collyres, des solutions de rinçage des lentilles de contact ou des conditionneurs pour des implants intraoculaires. Les solutions salines sont également couramment utilisées en ORL pour le lavage des fosses nasales.

D'autres compositions tentent de se rapprocher plus précisément de l'eau de mer. Ainsi, la demande FR 2 803 205 décrit une solution aqueuse à base d'eau de mer dont la teneur de certains ingrédients est modifiée par électrodialyse sélective. Une diminution du pH, une osmolalité inférieure, et des concentrations respectivement supérieures en potassium et inférieures en sodium, magnésium, calcium et chlore par rapport à celles de l'eau de mer, permettent une utilisation pour l'hygiène de l'oeil.

La demande FR 2 843 029 décrit une solution de rinçage des lentilles de contact à base d'eau de mer. Certaines caractéristiques de ses constituants peuvent être modifiées ; ainsi son pH et son osmolalité peuvent être inférieurs à ceux de l'eau de mer naturelle. A ces modifications peuvent s'ajouter une concentration supérieure en potassium et une concentration inférieure en sodium, magnésium, calcium et chlore.

De telles solutions d'eau de mer traitées de l'état de la technique, ne permettent pas de répondre sélectivement à certains besoins, en particulier pour ce qui concerne les thérapies de l'oeil et des muqueuses et, plus particulièrement, leur efficacité reste souvent très limitée pour le traitement des pathologies impliquant une interaction avec les récepteurs P2X tels que P2X7.

Pour pallier tout ou partie des inconvénients de l'état de la technique, la présente invention a pour objet une composition aqueuse obtenue à partir d'eau purifiée, de pH de 5.5 à 7.5, d'osmolalité de 200 à 400 mOsm/kg, de conductivité de 17 à 22.5 mS/cm à 25°C et préparée à partir de 1 à 20% d'eau de mer filtrée, et telle que ladite composition incorpore un sel de potassium dans une teneur de 90 à 180 mmol/L ainsi que du Zn(X)₂ à une concentration de 200 à 300 micromoles/L. Les concentrations sont données en référence à la composition finale dans laquelle le sel est ajouté, c'est également le cas pour l'ensemble des concentrations décrites dans la suite de la présente demande (sauf si une autre référence est expressément mentionnée). L'eau de mer est considérée comme le réservoir de tous les oligo-éléments dans la mesure où ils sont quasiment tous représentés, dans des concentrations plus ou moins importantes.

De préférence, la composition selon l'invention incorpore une teneur de sel de potassium inférieure à 150 mmol/L et avantageusement de 95 à 145 mmol/L, et plus avantageusement la teneur en sel de potassium est de 98 à 141 mmol/L.

L'osmolalité est mesurée selon la méthode décrite dans la Pharmacopée Européenne 6.0 (sixième édition) - point 2.2.35. La conductivité est mesurée selon la méthode décrite dans la Pharmacopée Européenne 6.0 - point 2.2.38. L'osmolalité de la solution est avantageusement de 250 à 350 mOsm/kg, voire de 270 à 330 mOsm/kg.

Le tableau 1 montre les éléments constitutifs de l'eau de mer ainsi que leur proportion théorique, exprimée en parts par million :

**Tableau 1 :**

| **Element** | **ppm** |
|---|---|
| Chlorine, Cl | 19 500 |
| Sodium, Na | 10 770 |
| Magnesium, Mg | 1 290 |
| Sulfure, S | 905 |
| Calcium, Ca | 412 |
| Potassium, K | 380 |
| Bromine, Br | 67 |
| Carbone, C | 28 |
| Zirconium, Zr | 0.00003 |
| Bismuth, Bi | 0.00002 |
| Niobium, Nb | 0.00001 |
| Thallium, Tl | 0.00001 |
| Thorium, Th | 0.00001 |
| Hafnium, Hf | 7 x 10⁻⁶ |
| Hélium, He | 6.8 x 10⁻⁶ |
| Béryllium, Be | 5.6 x 10⁻⁶ |
| Nitrogen, N | 11.5 |
| Strontium, Sr | 8 |
| Oxygen, O | 6 |
| Boron, B | 4.4 |
| Silicon, Si | 2 |
| Fluorine, F | 1.3 |
| Argon, Ar | 0.43 |
| Lithium, Li | 0.18 |
| Rubidium, Rb | 0.12 |
| Phosphorus, P | 0.06 |
| Iodine, I | 0.06 |
| Barium, Ba | 0.02 |
| Molybdenium, Mo | 0.01 |
| Arsenic, As | 0.0037 |
| Uranium, U | 0.0032 |
| Vanadium, V | 0.0025 |
| Titanium, Ti | 0.001 |
| Zinc, Zn | 0.0005 |
| Nickel, Ni | 0.00048 |
| Aluminium, Al | 0.0004 |
| Cesium, Cs | 0.0004 |
| Chromium, Cr | 0.0003 |
| Antimony, Sb | 0.00024 |
| Krypton, Kr | 0.0002 |
| Selenium, Se | 0.0002 |
| Neon, Ne | 0.00012 |
| Manganese, Mn | 0.0001 |
| Cadmium, Cd | 0.0001 |
| Copper, Cu | 0.0001 |
| Tungsten, W | 0.0001 |
| Iron, Fe | 0.000055 |
| Xenon, Xe | 0.00005 |
| Germanium, Ge | 5 x 10⁻⁶ |
| Gold, Au | 4 x 10⁻⁶ |
| Rhenium, Re | 4 x 10⁻⁶ |
| Cobalt, Co | 3 x 10⁻⁶ |
| Lanthanum, La | 3 x 10⁻⁶ |
| Neodymium, Nd | 3 x 10⁻⁶ |
| Lead, Pb | 2 x 10⁻⁶ |
| Silver, Ag | 2 x 10⁻⁶ |
| Tantalum, Ta | 2 x 10⁻⁶ |
| Gallium, Ga | 2 x 10⁻⁶ |
| Yttrium, Y | 1.3 x 10⁻⁶ |
| Mercury, Hg | 1 x 10⁻⁶ |
| Cerium, Ce | 1 x 10⁻⁶ |
| Dysprosium, Dy | 9 x 10⁻⁷ |
| Erbium, Er | 8 x 10⁻⁷ |
| Ytterbium, Yb | 8 x 10⁻⁷ |
| Gadolinium, Gd | 7 x 10⁻⁷ |
| Praseodymium, Pr | 6 x 10⁻⁷ |
| Scandium, Sc | 6 x 10⁻⁷ |
| Tin, Sn | 6 x 10⁻⁷ |
| Holmium, Ho | 2 x 10⁻⁷ |
| Lutetium, Lu | 2 x 10⁻⁷ |
| Thulium, Tm | 2 x 10⁻⁷ |
| Indium, In | 1 x 10⁻⁷ |
| Trebium, Tb | 1 x 10⁻⁷ |
| Palladium, Pd | 5 x 10⁻⁸ |
| Samarium, Sm | 5 x 10⁻⁸ |
| Tellurium, Te | 1 x 10⁻⁸ |
| Europium, Eu | 1 x 10⁻⁸ |
| Radium, Ra | 7 x 10⁻¹¹ |
| Protactinium, Pa | 5 x 10⁻¹¹ |
| Radon, Rn | 6 x 10⁻¹⁶ |

On voit, d'après ce tableau 1, que de nombreux éléments chimiques sont représentés, en quantité plus ou moins importante, dans l'eau de mer. Ces concentrations sont exprimées en valeurs moyennes, qui peuvent varier selon la localisation géographique de la mer, et même du lieu de pompage de l'eau de mer.

L'eau de mer destinée à la composition selon l'invention est pompée directement en mer de manière à respecter son état initial, et par conséquent ses propriétés intrinsèques. La concentration des éléments constitutifs de l'eau peut varier en fonction de la localisation géographique du lieu de pompage, et nécessiter éventuellement une modification de la concentration en certains éléments. Le lieu de pompage sera donc choisi avec précision de manière à ce que l'eau puisse être utilisée directement, sans modification des concentrations de ses éléments constitutifs.

Ainsi, par exemple, l'eau de mer sera choisie de manière à ne pas avoir une concentration en sodium trop importante, dans la mesure où cet élément est impliqué dans les réactions inflammatoires quand il est présent en quantité trop importante.

L'eau de mer utilisée dans le cadre de l'invention a un pH de 7.5 à 8.5, comprenant une concentration en sels ou ions proche de la composition théorique de l'eau de mer (tableau 1).
Les eaux de mer utilisées sont sélectionnées pour leurs emplacements privilégiés :
- absence d'industries à proximité, avec un environnement sain,
- site de prélèvement non enclavé : renouvellement constant d'eau,
- courants sur la zone favorisant le renouvellement de l'eau,
- qualité reconnue satisfaisante par des organismes spécialisés, tels que l'IFREMER et la DDASS.

Les inventeurs ont découvert, de manière tout à fait inattendue, qu'une solution d'eau de mer diluée dans la plage de valeur conformément à la composition selon la présente invention, en combinaison avec un ajout de sel de zinc dans les teneurs préconisées, permettait après application sur l'oeil, de par une synergie d'action, d'obtenir une réponse en créant un signal électrique spécifique apte à agir sur les canaux ioniques des cellules de l'oeil. Il a été mis en évidence que la composition selon l'invention, notamment ses ions, permet un transport passif et actif vers les cellules, dû à ses canaux ioniques.

La composition selon l'invention a montré, de manière surprenante qu'elle présente une grande tolérance pour les tissus les plus fragiles (réduction de stress oxydatif ou inflammatoire...), telle que la surface oculaire et les muqueuses et une activité anti-apoptotique (par action sur le récepteur P2X7). Elle peut être utilisée pour des applications directes (rinçage) ou comme excipient (cosmétiques, dispositifs médicaux...). L'étude de la cytotoxicité de la solution selon l'invention a permis d'évaluer sa haute tolérance pour son utilisation en ophtalmologie. Différents tests d'évaluation de la cytotoxicité ont été réalisés pour cerner au mieux la tolérance de la composition selon l'invention par les différents types de cellules de la surface oculaire (cornéennes et conjonctivales).

L'eau de mer à la base de la composition selon l'invention, constitue un réservoir de différents éléments chimiques, présents en concentration plus ou moins importante. Les propriétés physico-chimiques de l'eau de mer sont dues à un équilibre entre anions et cations. Ces éléments se comportent comme un ensemble de biomolécules interagissant les unes avec les autres. Dans le cadre de l'invention, la synergie d'action se fait non seulement entre ces différents ions, mais également avec les sels de zinc présents dans la composition.

L'eau de mer est diluée et enrichie en zinc puis est ajustée afin d'obtenir une solution dans la gamme de pH et d'osmolalité souhaitée, par ajout d'un sel. Le sel utilisé préférentiellement est le chlorure de potassium. L'interaction des ions zinc avec les différents éléments de l'eau de mer renforce son action sur les stress cellulaires.

De préférence, le pH de la composition est de 6 à 7.2.

Une étude a été réalisée par la demanderesse sur les récepteurs de mort cellulaire P2X. Ces récepteurs sont des récepteurs ionotropiques conduisant un influx cationique après leur activation. Plus précisément, parmi les récepteur P2X cités ci-dessus, le récepteur P2X7 induit des réponses inflammatoires, une surproduction des espèces réactives de l'oxygène, une apoptose, voire une cytolyse. Les différents essais comparatifs montrent, comme cela est décrit dans la partie expérimentale, que l'activation des récepteurs P2X7 est fortement inhibée en présence d'une composition à base d'eau de mer selon l'invention. L'inhibition de l'activation des récepteurs P2X7 peut être assimilée à un ordre de « non-mort » des cellules, atténuant les phénomènes inflammatoires et l'apoptose. Cela démontre également une bonne tolérance cellulaire.

La composition d'eau de mer est enrichie en zinc, et plus particulièrement en un sel du type Zn(X)₂ à une concentration de 125 à 500 µmol/L, X⁻ pouvant être tout type de contre-anion complexé avec le cation Zn²⁺.

Comme source de Zinc, on préfère incorporer dans la composition selon l'invention du chlorure de zinc (ZnCl₂), favorisant les interactions avec les autres éléments constitutifs de l'eau de mer. Dans ce cadre, la présente invention concerne avantageusement une composition telle que décrite précédemment, pour lequel le sel de zinc est un chlorure de zinc (ZnCl₂).

De préférence, le Zn(X)₂ est incorporé dans une teneur de 200 à 300 µmol/L.

La composition selon l'invention comporte un sel de potassium qui est de préférence un chlorure de potassium (KCl).

L'enrichissement de l'eau de mer avec d'autres ions, tels que Mg²⁺ ou Ca²⁺ ne donne pas de résultats aussi probants que ceux obtenus avec le Zn²⁺.

De préférence, l'eau de mer est présente dans la composition dans une teneur de 3 à 17 %.

La composition peut être utilisée comme excipient ou comme produit de contact selon sa destination.

La composition selon l'invention est avantageusement destinée à une utilisation comme médicament dans le traitement des maladies oculaires par inactivation des récepteurs de mort cellulaire du type P2X7.

La composition selon l'invention peut également être utilisée en tant que collyre, larmes artificielles, solution de rinçage pour lentilles, solution de lavage oculaire et/ou comme produit de récipient pour implant oculaire. En raison de sa polarité, elle possède de bonnes propriétés lavantes ainsi que de bonnes propriétés de solvant. De ce fait, elle permet le rinçage de l'oeil en cas de projection de produits nocifs, sans provoquer d'irritation et favorisant la régénération cellulaire.

La composition selon l'invention a été décrite précisément pour l'ophtalmologie, mais pourrait s'appliquer néanmoins à tout autre domaine faisant intervenir la physiologie cellulaire, dans d'autres organes tels que les organes de la sphère ORL - notamment les muqueuses nasales - en vu de prévenir les affections telles que les rhinites, les congestions nasales, les sécheresses nasales, les sinusites ou les bronchites. La composition selon l'invention peut également s'appliquer à la peau et aux muqueuses vaginales, notamment dans des préparations pour l'hygiène intime. La composition selon l'invention est destinée avantageusement pour une utilisation comme médicament dans le traitement des muqueuses nasales, buccales, vaginales et/ou de la peau.

Aussi, pour nettoyer des tissus agressés, des muqueuses ou la peau, la composition selon l'invention peut stimuler la cicatrisation, grâce à ses propriétés. La composition selon l'invention est destinée avantageusement pour une utilisation comme médicament dans le traitement de la cicatrisation des muqueuses et/ou de la peau.

La composition selon l'invention peut également être utilisée en tant que spray, goutte ou excipient de solubilisation de molécules présentant un intérêt thérapeutique telles que l'acide hyaluronique, les polyphénols ou les acides gras...

La présente invention concerne aussi un procédé de préparation d'une composition telle que décrite précédemment dans le cadre de l'invention, comprenant les étapes :
a) pomper l'eau de mer ;
b) prétraiter éventuellement l'eau de mer à l'aide d'un premier filtre naturel ou artificiel ;
c) filtrer l'eau de mer éventuellement prétraitée au travers d'un deuxième filtre dont le diamètre moyen des pores est inférieur ou égal 5 µm;
d) effectuer une autre filtration au travers d'un troisième filtre dont le diamètre moyen des pores est inférieur à ceux du deuxième filtre et est inférieur ou égal 0.45 µm;
e) diluer la solution filtrée avec de l'eau purifiée.

A l'étape a), l'eau de mer prélevée par pompage est filtrée éventuellement (étape b)) à l'aide, soit d'un filtre naturel comme par exemple du sable, soit d'un filtre artificiel comme par exemple une grille adaptée pour retirer les particules de grosse taille. L'eau est ensuite avantageusement entreposée dans un bassin de prétraitement. Cette eau de mer est ensuite redirigée vers des canalisations à l'aide d'une pompe en vue de sa filtration.

L'eau de mer prétraitée est filtrée aux étapes c) et d) au travers de deux filtres dont les diamètres moyens sont respectivement de 5 µm à 1 µm (pour c)), et inférieur à 0,45 µM (pour d)). Pour cette étape de filtration, l'eau est de préférence injectée dans un ensemble de filtration intégrant des canalisations qui comportent avantageusement des filtres sous forme de cartouches cylindriques. Un exemple d'ensemble de filtration est décrit à la suite dans la partie expérimentale.

La présente invention sera mieux comprise à la lecture de la partie expérimentale qui va suivre, dont les exemples et les figures sont uniquement donnés à titre illustratif et ne peuvent nullement être considérés comme limitatif. Pour illustrer les résultats de la partie expérimentale, le schéma 1 représente une vue schématique en coupe transversale d'un ensemble de filtration qui permet de filtrer l'eau de mer avant sa dilution pour obtenir la composition selon l'invention.

### PARTIE EXPERIMENTALE

L'osmolalité est mesurée selon la méthode décrite dans la Pharmacopée Européenne 6.0 (sixième édition) - point 2.2.35. La conductivité est mesurée selon la méthode décrite dans la Pharmacopée Européenne 6.0 - point 2.2.38.

### 1) Préparation d'une composition selon l'invention comprenant 5 % d'eau de mer (SCHEM. 1) :

5 Litres d'une eau de mer prélevée dans le sud du Finistère par pompage sont filtrées à l'aide d'une trémie. L'eau de mer ainsi prélevée est entreposée dans un bassin de prétraitement. On procède ensuite au pompage de cette eau de mer à l'aide d'une pompe.

L'eau de mer prétraitée est injectée dans les canalisations d'un ensemble de filtration 1 représenté sur le schéma 1. L'ensemble de filtration 1 comprend une arrivée 2 de l'eau de mer. Cette arrivée d'eau 2 est connectée à un débitmètre 3 qui est branché sur la partie basse du carter 4 d'un préfiltre 5 (1 µm). La sortie du préfiltre 5 (toujours en partie basse du carter 4) est branchée sur la partie basse du carter 6 d'un filtre 7 (0.22 µm) dont la sortie est reliée à une canalisation qui sert à collecter l'eau issue de la filtration pour la stocker dans un container 8.

On complète ensuite la solution filtrée avec de l'eau distillée et on ajoute du chlorure de zinc et du chlorure de potassium de sorte à obtenir une composition (1) comportant 250 µmol/L de ZnCl₂ et 140.9 mmol/L de KCl. La conductivité de la solution est de 20 mS/cm à 25 °C, et l'Osmolalité est de 300 mOsmol/Kg.

### 2) Etude de la viabilité cellulaire :

Des cellules WKD (cellules épithéliales conjonctivales humaines) sont mises à incuber dans différents milieux de culture et la viabilité cellulaire est évaluée par le test au rouge neutre. Le rouge neutre est incubé pendant 3 heures à 37°C avec les cellules puis les cellules sont rincées avec du tampon PBS et une solution d'acide acétique/éthanol est utilisée pour libérer le rouge neutre des cellules et homogénéiser la coloration. L'appareil utilisé pour quantifier la fluorescence est un cytomètre adapté aux microplaques (Safire, TECAN). Le test au rouge neutre est recommandé pour l'évaluation de la cytotoxicité des dispositifs médicaux dans la norme ISO 10993-5 version de juillet 2010. Les résultats de ce test sont exprimés en pourcentage par rapport aux cellules contrôles.

Les résultats sont reportés dans le tableau 2.

**Tableau 2.**

| Compositions utilisées pour l'incubation des cellules | Solution PBS | Eau de mer 5% | Eau de mer 5% + KCl 140.9 mM | Eau de mer 5% + ZnCl₂ 250 µmol/L | Eau de mer 5% + KCl 140.9 mM + ZnCl₂ 250 µmol/L | Eau de mer 5% + KCl 100 mM + ZnCl₂ 250 µmol/L | Eau de mer 5% + KCl 160 mM + ZnCl₂ 250 µmol/L | Eau de mer 5% + KCl 250 mM + ZnCl₂ 250 µmol/L | Eau de mer 5% + KCl 500 mM + ZnCl₂ 250 µmol/L |
|---|---|---|---|---|---|---|---|---|---|
| Pourcentage de cellules vivantes après 1 h d'incubation | 100 | 54 | 102 | 42 | 97 | 102 | 63 | 43 | 25 |

On constate que la composition comportant 5% d'eau de mer et du KCI à 140.9 mmol/L montre une tolérance cellulaire (102%) améliorée par rapport à la composition équivalente ne contenant que l'eau de mer, et, qu'en outre cette tolérance n'est pas dégradée par la présence de ZnCl₂ à raison de 250 µmol/L.

### 3) Mesure de l'état d'oxvdo-réduction des cellules :

La composition préparée à la partie 1) (désignée ci-après composition (1)) a été testée en vue d'évaluer son pouvoir cytotoxique. Un agent cytotoxique a le pouvoir de baisser le potentiel redox d'une cellule. Cela provoque la diminution de l'activité des enzymes d'oxydo-réduction et donc la mort cellulaire.

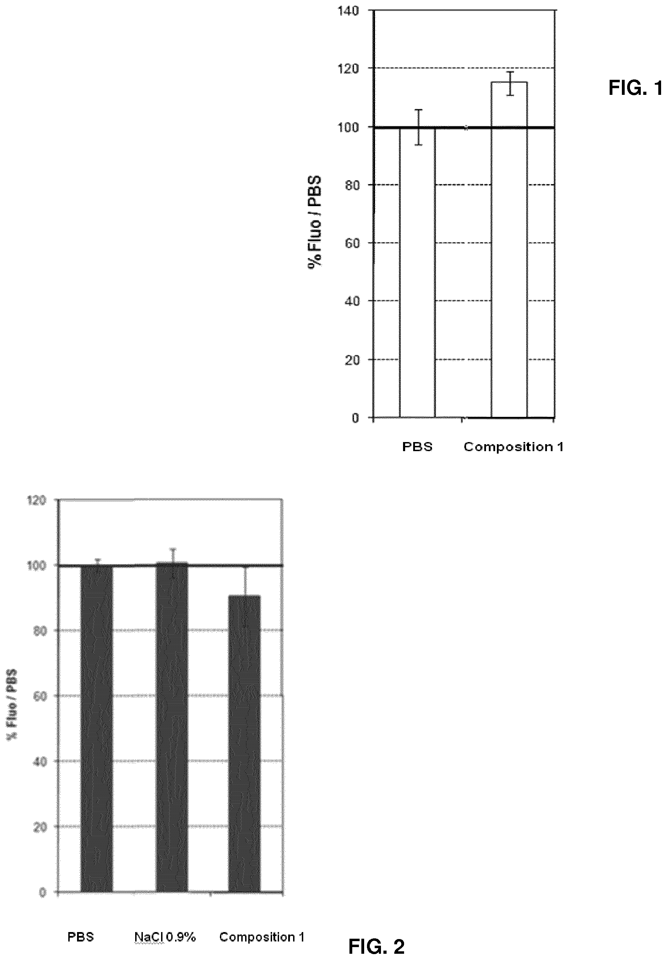

Dans le test effectué, on observe une sonde fluorescente, l'alamar blue, incubée pendant 6 heures à 37°C avec les cellules. L'appareil utilisé pour quantifier la fluorescence est un cytomètre adapté aux microplaques (Safire, TECAN). Ce test fait partie des tests proposés pour l'évaluation du potentiel cytotoxique oculaire des solutions multifonction pour l'entretien des lentilles de contact dans la norme NF S11-820 dans sa version de février 2012. L'intensité de la fluorescence est proportionnelle à la capacité d'oxydo-réduction, et par extension aux nombres de cellules vivantes. Les tests sont réalisés en comparaison avec une solution de NaCl à 0.9 % et une solution tampon phosphate salin (PBS, couramment utilisé en culture cellulaire), et les résultats sont reportés sur les figures 1 et 2. Les résultats montrent en ordonnée le pourcentage de fluorescence quantifié par rapport au PBS (ou «% Fluo / PBS»). Ce ratio est calculé en divisant l'intensité de fluorescence des cellules traitées par l'intensité de fluorescence des cellules non traitées (incubées avec le tampon PBS), puis on multiplie par 100 pour obtenir un pourcentage. Pour la figure 1, les résultats concernent une incubation d'une heure sur des cellules WKD (cellules épithéliales conjonctivales humaines), et pour la figure 2, ils concernent une incubation d'une heure sur des cellules HCE (cellules épithéliales cornéennes humaines).

Les résultats montrent que la composition n'induit pas de diminution du potentiel redox par rapport au témoin sur cellules de la conjonctive.

On observe une légère diminution de fluorescence pour les cellules de la cornée mais celle-ci n'est pas statistiquement significative.

En conclusion, l'incubation dans la composition (1) ne crée pas de perte du potentiel redox, il n'y a donc pas de diminution du nombre de cellules vivantes. La composition 1 est donc bien tolérée.

### 4) Modulation de l'activation des P2X7 :

Des cellules WKD (cellules épithéliales conjonctivales humaines) sont mises à incuber dans différentes solutions salines et l'activation des récepteurs P2X7 est quantifiée grâce au test YO-PRO-1. La sonde YO-PRO-1 entre dans les cellules suite à l'ouverture d'un pore membranaire, issue de l'activation du récepteur P2X7. Une augmentation de la fluorescence correspond à une activation du récepteur P2X7. L'activation du P2X7 est signe d'apoptose. L'appareil utilisé pour quantifier la fluorescence est un cytomètre adapté aux microplaques (Safire, TECAN).

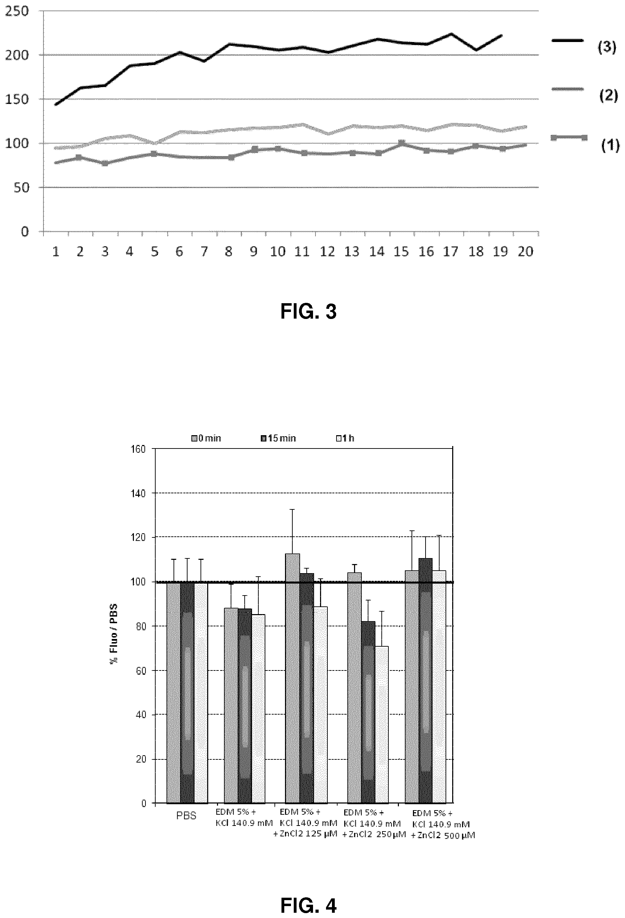

A la lecture de la figure 3 (en abscisse, on lit le temps en minutes, et les ordonnées sont à lire en unité RFU ou unité relative de fluorescence), on constate que la composition (2) comportant 5% d'eau de mer et du KCI à 140.9 mmol/L montre une inhibition de l'activation des P2X7, cette inhibition est visible par rapport à une composition (3) de référence de NaCl à 0.9%, et, qu'en outre, cette inhibition est encore améliorée par la présence de ZnCl₂ à raison de 250 µmol/L pour la composition (1) (pour mémoire, la composition (1) comporte 5% d'eau de mer, du KCI à 140.9 mmol/L et du ZnCl₂ à 250 µmol/L).

### 5) Inhibition de la production d'espèces réactives de l'oxygène :

On mesure la production d'espèces réactives de l'oxygène (ERO), sur les cellules WKD grâce au test H2DCF-DA. Cette sonde entre dans les cellules et est hydrolysée par les enzymes intracellulaires. Une fois hydrolysée, la sonde est piégée dans les cellules et peut être oxydée par les ERO présentes. Cette forme oxydée est alors fluorescente. L'appareil utilisé pour quantifier la fluorescence est un cytomètre adapté aux microplaques (Safire, TECAN).

Les tests sont réalisés en se référant à une solution tampon phosphate salin (PBS, couramment utilisé en culture cellulaire), et les résultats sont reportés à la figure 4. Les résultats montrent en ordonnée le pourcentage de fluorescence quantifié par rapport au PBS. Ce ratio est calculé en divisant l'intensité de fluorescence des cellules traitées par l'intensité de fluorescence des cellules non traitées (incubées avec le tampon PBS), puis on multiplie par 100 pour obtenir un pourcentage.

On constate, à la lecture de la figure 4, que pour les solutions suivantes les commentaires suivants s'appliquent :
> Solution d'eau de mer (ou « EDM ») 5% + KCI à 140.9 mM : il y a inhibition du stress oxydatif ; et
> Solution d'eau de mer 5% + KCl de 140.9 mM + 250 µM ZnCl₂: l'inhibition du stress oxydatif est optimale.

Pour les plus forts taux de ZnCl₂, ou les taux plus faibles, l'inhibition est moins franche voire inexistante.

### 6) Evaluation de l'intérêt de la composition (1) dans le rinçage de l'oeil après exposition à un agent toxique :

Lors d'un travail *in vitro,* l'impact du rinçage par différentes solutions d'un oeil brûlé chimiquement, a été étudié. (Said et al. 2009)

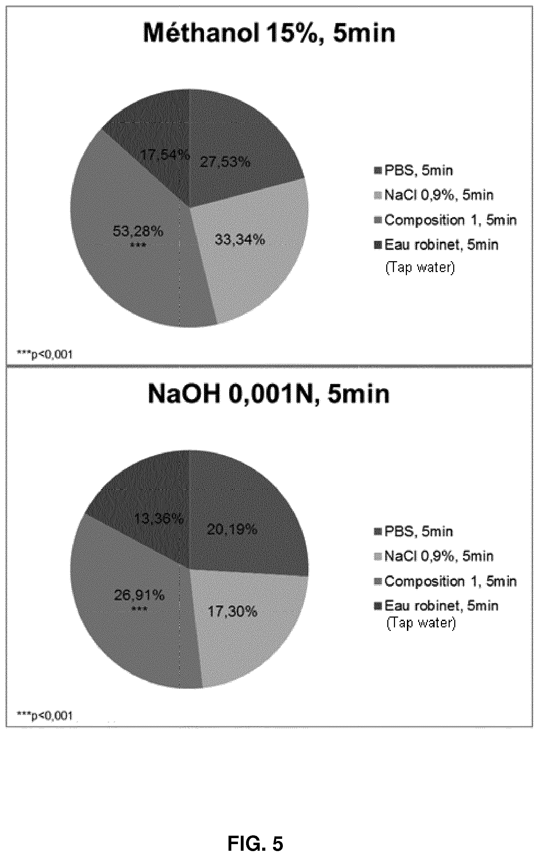

Une évaluation de la viabilité cellulaire par le test au rouge neutre 24 h après la brûlure au méthanol ou à la soude, suivie de l'application des solutions de rinçage a été réalisée. Le rouge neutre est incubé pendant 3 heures à 37°C avec les cellules puis les cellules sont rincées avec du tampon PBS et une solution d'acide acétique/éthanol est utilisée pour libérer le rouge neutre des cellules et homogénéiser la coloration. L'appareil utilisé pour quantifier la fluorescence est un cytomètre adapté aux microplaques (Safire, TECAN). Le test au rouge neutre est recommandé pour l'évaluation de la cytotoxicité des dispositifs médicaux dans la norme ISO 10993-5 dans sa version de juillet 2010. Les résultats de ce test évalué en pourcentage par rapport aux cellules contrôles, sont présentés à la figure 5 (p<0.001 est indiqué par rapport aux autres compositions réparties sur le camembert).

On constate que le rinçage par la composition (1) améliore la survie des cellules et la récupération cellulaire après brûlure chimique. Cette amélioration est statistiquement significative par rapport aux autres solutions utilisées (dont le NaCl 0,9%).

Une étude a été réalisée sur des lapins. Le tableau 3, présenté ci-après, répertorie les résultats des tests de Draize réalisés après brûlure oculaire par un produit chimique basique puis rinçage avec les différentes solutions. Dans ce test, l'application d'un produit cicatrisant (Euronac®) a également été ajoutée après le rinçage avec la solution de NaCl à 0.9% ou la composition (1) selon l'invention.

### Tableau 3 :

| | NaCl 0.9% | NaCl 0.9% + Euronac® | Composition 1 + Euronac® |
|---|---|---|---|
| 1 | 100 | 100 | 96 |
| 24 | 108 | 102 | 98 |
| 48 | 104 | 100 | 94 |
| 144 | 54 | 50 | 20 |
| 168 | 52 | 50 | 10 |
| 192 | 52 | 50 | 10 |

La première ligne de ce tableau indique les différents traitements, et la première colonne indique le temps en heures.

Plus le score est faible, plus l'irritation est faible. On peut voir que le rinçage au NaCl 0,9% avant application d'Euronac® commercialisé par les laboratoires Doliage (collyre cicatrisant) n'abaisse pas le score en-dessous de 50. Tandis que la solution selon l'invention abaisse le score à 20 puis à 10 en une semaine (remarque : un score inférieur à 15 indique l'absence d'irritation).

Les résultats de cette étude mettent en avant un meilleur pronostic lorsque l'oeil est rincé par la composition (1) à la place d'une solution de type NaCl 0,9% avant application du collyre cicatrisant.

### 7) Etude du pouvoir rinçant de produits d'entretien de lentilles de contact :

Une pré-étude a été réalisée sur des lentilles rigides décontaminées par une solution d'acide peracétique et peroxyde d'hydrogène. Lors de cette pré-étude, nous avions constaté que la composition (1) permettait un rinçage efficace de cette solution.

Nous avons alors étendu les investigations au rinçage des solutions multifonctions utilisées pour entretenir les lentilles de contact souples hydrophiles.

Une première série d'essais a été réalisée de la manière suivante :
- 3 types de lentilles ont été exposés à différentes solutions multifonctions,
- la viabilité cellulaire après application des lentilles sur un tapis de cellules cornéennes a été observée au microscope et par mesure au rouge neutre,
- l'expérience a été renouvelée après un rinçage des lentilles par la solution selon l'invention.

Le rinçage par le NaCl 0.9% et la composition 1 selon l'invention ont été comparés.

Le tableau 4, montré ci-après résume les résultats de viabilité cellulaire obtenus :

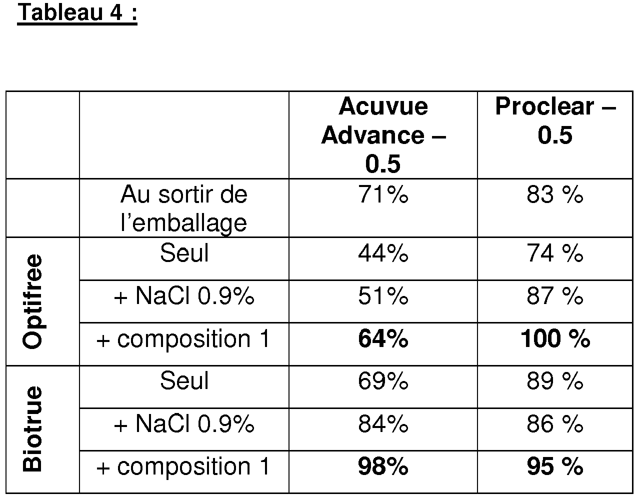

Différents types de lentilles et de solutions multifonctions d'entretien de lentilles de contact (SME) (Biotrue et Optifree) ont été testés. La solution Biotrue est commercialisée par Bausch&Lomb et contient de l'acide hyaluronique, de la sulfobétaïne, du poloxamine, de l'acide borique, du borate de sodium, de l'édétate de sodium, du polyaminopropyl biguanide, du polyquaternium et du chlorure de sodium ; la solution Optifree est commercialisée par Alcon et contient entre autres du Tetronic 1304, du Polyquad®, de l'Aldox®, un tampon citrate, du chlorure de sodium, de l'édétate de sodium. Ces deux solutions sont commercialisées pour l'entretien des lentilles de contact souples.
Le rinçage par le NaCl 0.9% et la composition (1) ont été comparés. Les lentilles sont trempées 96h dans 100 ml d'une SME (= 20 cycles d'entretien, protocole de la Food and Drug Administration de 1994 FDA Premarket Notification 510(k) Guidance Document for Daily Wear Contact Lens), puis rincées par trempage 24h dans 100 ml de la composition 1 ou NaCl 0.9%.

Dans tous les cas, le rinçage réduit la perte de viabilité cellulaire.

Une amélioration notable est constatée grâce au rinçage des lentilles de contact. La composition (1) selon l'invention apporte une meilleure viabilité cellulaire.

### 8) Etude de la protection contre l'inflammation sur des macrophages

On mesure le relargage de cytokines pro-inflammatoires (TNF-α) sur les macrophages par la technique ELISA (Enzyme-Linked Immunosorbent Assay). Cette technique permet le dosage de protéines dans un échantillon ; dans notre cas, la technique ELISA nous permet de quantifier la cytokine TNF-α, libérée dans le surnageant cellulaire par les macrophages, suite à un stress inflammatoire. L'antigène spécifique du TNF-α est accroché à un support, une microplaque 96 puits. On y dépose le surnageant cellulaire et le TNF-α peut se lier à son antigène. Un anticorps primaire permet de reconnaître le TNF-α, puis un anticorps secondaire conjugué à une enzyme se lie à l'anticorps primaire. Une réaction enzymatique colorimétrique permet la révélation du complexe anticorps. L'appareil utilisé pour quantifier l'absorbance est un cytomètre adapté aux microplaques (Safire, TECAN). En parallèle, une gamme étalon est réalisée à partir d'une solution de TNF-α pour permettre de transposer l'absorbance en quantité de protéine (pg/mL).

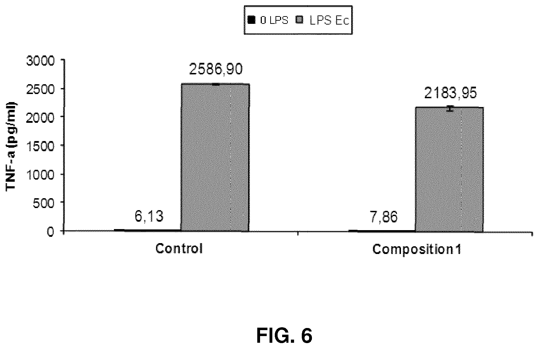

Les tests sont réalisés en se référant au milieu de culture, et les résultats sont reportés à la figure 6.

Les résultats montrent en ordonnée la quantité de TNF-α dosée dans le surnageant des macrophages en pg/mL. Les macrophages ont été incubés avec la composition 1 diluée à 1:2 dans le milieu de culture puis stimulés avec du LPS (lipopolysaccharide) d'Escherichia Coli pendant 24 heures à 37°C.

La figure 6 montre les résultats obtenus pour le contrôle (ou control.) - sans (ou « 0 LPS ») et avec le LPS d'Escherichia Coli. La figure 6 montre également les résultats obtenus pour la composition (1) sans et avec le LPS d'Escherichia Coli. D'après la figure 6, la composition (1) est favorable à la réduction des phénomènes inflammatoires.

Le tableau 5 reprend les résultats de la figure 6 pour les exprimer sous forme de ratio : la quantité de TNF-α sécrétée dans le contrôle sans LPS est rapportée à 1, et les quantités de TNF-α sécrétées dans les autres conditions sont toutes rapportées à la quantité de TNF-α sécrétée dans le contrôle (en divisant la quantité de TNF-α par la quantité de TNF-α dans le contrôle).

**Tableau 5 :**

| | **Seul** | **LPS** |
|---|---|---|
| **Contrôle** | **1** | **422,0** |
| **Composition 1** | **1,28** | **356,27** |

Lorsque les cellules sont stimulées avec du LPS pour induire une inflammation, la préincubation avec la composition 1 diluée à 1:2 réduit la production de TNFa (x422 → x356).

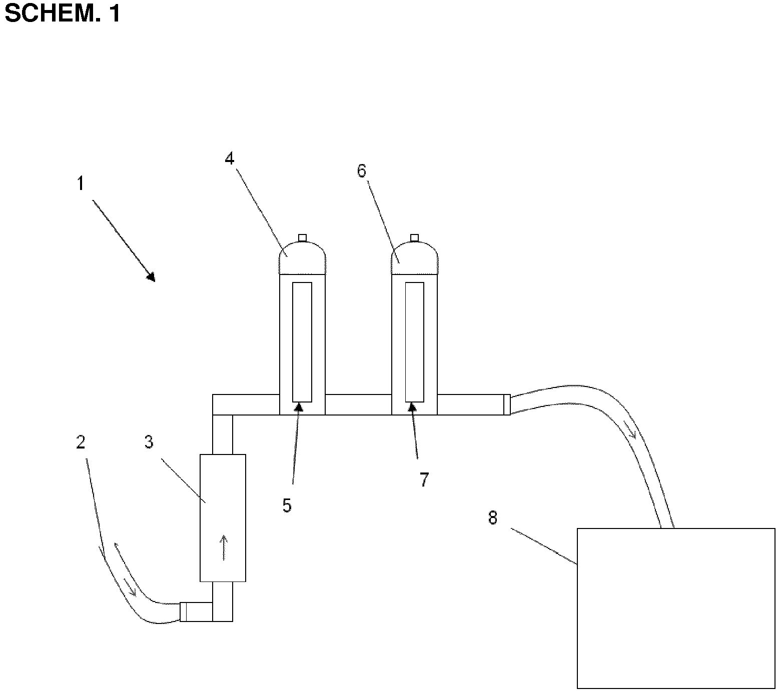

## Revendications

1. Composition aqueuse obtenue à partir d'eau purifiée, de pH de 5.5 à 7.5, d'osmolalité de 200 à 400 mOsm/kg, de conductivité de 17 à 22.5 mS/cm à 25°C et préparée à partir de 1 à 20% d'eau de mer filtrée, ladite composition incorpore un sel de potassium dans une teneur de 90 à 180 mmol/L, et ladite composition incorpore en outre du Zn(X)₂ à une concentration de 200 à 300 µmol/L.

2. Composition selon la revendication 1, **caractérisée en ce que** le pH est de 6 à 7.2.

3. Composition selon l'une des revendications 1 ou 2, **caractérisée en ce que** les ions X⁻ sont des ions chlorures.

4. Composition selon l'une des revendications 1 à 3, **caractérisée en ce que** le sel de potassium est du chlorure de potassium.

5. Utilisation de la composition selon l'une des revendications 1 à 4, comme solution de rinçage pour lentilles ou comme produit de récipient pour implant oculaire.

6. Composition selon l'une des revendications 1 à 4, pour son utilisation comme médicament dans le traitement des maladies oculaires par inactivation des récepteurs de mort cellulaire du type P2X7.

7. Composition pour utilisation selon la revendications 6, la composition étant sous forme de collyre, larmes artificielles ou solution de lavage oculaire

8. Composition selon l'une des revendications 1 à 4, pour son utilisation comme médicament dans le traitement d'au moins l'un des éléments sélectionné parmi les muqueuses nasales, les muqueuses buccales, les muqueuses vaginales et la peau.

9. Composition selon l'une des revendications 1 à 4, pour son utilisation comme médicament dans le traitement de la cicatrisation d'au moins l'un des éléments sélectionné parmi des muqueuses et de la peau.

10. Utilisation de la composition selon l'une des revendications 1 à 4, comme excipient de solubilisation de molécules présentant un intérêt thérapeutique.

11. Procédé de préparation d'une composition selon l'une des revendications 1 à 4 comprenant les étapes :
a) pomper l'eau de mer ;
b) prétraiter éventuellement l'eau de mer à l'aide d'un premier filtre naturel ou artificiel ;
c) filtrer l'eau de mer éventuellement prétraitée au travers d'un deuxième filtre dont le diamètre moyen des pores est inférieur ou égal 5 µm;
d) effectuer une autre filtration au travers d'un troisième filtre dont le diamètre moyen des pores est inférieur à ceux du deuxième filtre et est inférieur ou égal 0.45 µm; et
e) diluer la solution filtrée avec de l'eau purifiée.

## Patentansprüche

1. Wässrige Zusammensetzung, die aus gereinigtem Wasser erhalten wird, mit einem pH-Wert von 5,5 bis 7,5, mit einer Osmolalität von 200 bis 400 mOsm/kg, mit einer Leitfähigkeit von 17 bis 22,5 mS/cm bei 25 °C, und hergestellt aus 1 bis 20 % gefiltertem Meerwasser, die Zusammensetzung ein Kaliumsalz in einem Gehalt von 90 bis 180 mmol/L beinhaltet, und die Zusammensetzung weiter Zn(X)₂ in einer Konzentration von 200 bis 300 µmol/L beinhaltet.

2. Zusammensetzung nach Anspruch 1, **dadurch gekennzeichnet, dass** der pH-Wert von 6 bis 7,2 beträgt.

3. Zusammensetzung nach einem der Ansprüche 1 oder 2, **dadurch gekennzeichnet, dass** die X⁻-Ionen Chloridionen sind.

4. Zusammensetzung nach einem der Ansprüche 1 bis 3, **dadurch gekennzeichnet, dass** das Kaliumsalz Kaliumchlorid ist.

5. Verwendung der Zusammensetzung nach einem der Ansprüche 1 bis 4 als Spüllösung für Linsen oder als Aufbewahrungsprodukt für Augenimplantate.

6. Zusammensetzung nach einem der Ansprüche 1 bis 4 zu deren Verwendung als Arzneimittel in der Behandlung der Augenerkrankungen durch Inaktivierung der Zelltod-Rezeptoren vom Typ P2X7.

7. Zusammensetzung zur Verwendung nach Anspruch 6, wobei die Zusammensetzung in Form von Augentropfen, künstlichen Tränen oder Augenwaschlösung vorliegt.

8. Zusammensetzung nach einem der Ansprüche 1 bis 4 zu deren Verwendung als Arzneimittel in der Behandlung von mindestens einem der Elemente, ausgewählt aus den Nasenschleimhäuten, den Mundschleimhäuten, den Vaginalschleimhäuten und der Haut.

9. Zusammensetzung nach einem der Ansprüche 1 bis 4 zu deren Verwendung als Arzneimittel in der Behandlung der Wundheilung von mindestens einem der Elemente, ausgewählt aus Schleimhäuten und Haut.

10. Verwendung der Zusammensetzung nach einem der Ansprüche 1 bis 4 als Hilfsstoff zur Solubilisierung von Molekülen, die einen therapeutischen Nutzen aufweisen.

11. Verfahren zur Herstellung einer Zusammensetzung nach einem der Ansprüche 1 bis 4, umfassend die Schritte:
a) Pumpen des Meerwassers;
b) gegebenenfalls Vorbehandeln des Meerwassers mithilfe eines ersten natürlichen oder künstlichen Filters;
c) Filtern des gegebenenfalls vorbehandelten Meerwassers durch einen zweiten Filter, dessen mittlerer Durchmesser der Poren kleiner oder gleich 5 µm ist;
d) Vornehmen einer weiteren Filterung durch einen dritten Filter, dessen mittlerer Durchmesser der Poren kleiner als jener des zweiten Filters ist, und kleiner oder gleich 0,45 µm ist; und
e) Verdünnen der gefilterten Lösung mit gereinigtem Wasser.

## Claims

1. Aqueous composition obtained from purified water with pH from 5.5 to 7.5, with osmolality from 200 to 400 mOsm/kg, with conductivity from 17 to 22.5 mS/cm at 25°C and prepared from 1 to 20% of filtered sea water, said composition incorporates a potassium salt with a content of 90 to 180 mmol/L and said composition also incorporates Zn(X)₂ at a concentration of between 200 and 300 µmol/L.

2. Composition according to claim 1, **characterised in that** the pH is 6 to 7.2

3. Composition according to either claim 1 or 2, **characterised in that** the X⁻ ions are chloride ions.

4. Composition according to one of claims 1 to 3, **characterised in that** the potassium salt is potassium chloride.

5. Use of the composition according to one of claims 1 to 4, as a rinsing solution for lenses or as a recipient product for an ocular implant.

6. Composition according to one of claims 1 to 4, for use as a medicine in the treatment of eye diseases by deactivation of P2X7 type dead cell receptors.

7. Composition for use according to claim 6, the composition being in the form of a collyrium, artificial tears or an eyewash solution.

8. Composition according to one of claims 1 to 4, for use as a medicine in the treatment of at least one element selected from among nasal, mouth and vaginal mucous membranes and the skin.

9. Composition according to one of claims 1 to 4, for use as a medicine in the treatment of healing of at least one element selected from among mucous membranes and the skin.

10. Composition according to one of claims 1 to 4, as an excipient for solubilisation of molecules with a therapeutic interest.

11. Method for preparation of a composition according to one of claims 1 to 4, comprising the following steps
a) pump sea water;
b) possibly pretreatment of sea water by means of a first natural or artificial filter;
c) filter the sea water, possibly pretreated, through a second filer for which the average pore diameter is less than or equal to 5 µm;
d) make another filtration through a third filter, in which the average pore diameter is less than the average pore diameter of the second filter and is less than or equal to 0.45 µm; and
e) dilute the filtered solution with purified water.
